# EUROPÄISCHE PATENTANMELDUNG

(11) **EP 1 044 647 A1**
(43) Veröffentlichungstag der Anmeldung: **18.10.2000**
(21) Anmeldenummer: 99810315.4
(22) Anmeldetag: 15.04.1999
(51) Int. Cl.: A61B 5/087, A61M 21/00

(54) **Atemtrainer**

(71) Anmelder: Baumann, Ulrich André, 3110 Münsingen (CH)
(72) Erfinder: Baumann, Ulrich André, 3110 Münsingen (CH)
(74) Vertreter: AMMANN PATENTANWAELTE AG BERN

(57) **Zusammenfassung**

Ein Gerät zur akustischen Rückkoppelung des Atemgeräusches auf ein Sinnesorgan derselben Person (1), insbesondere die Ohren (6), umfasst im wesentlichen einen Klangerzeuger in einem Gehäuse (10; 24), von dem aus geeignete Verbindungsmittel (4, 5), z.B. in der Art von Stethoskop-Hörschläuchen, zu den Ohren (6) abgehen. Als Klangerzeuger dienen Elemente, die durch den Atemluftstrom angeregt ein akustisches Signal erzeugen. Besonders geeignet hierfür sind Blätter, wie sie z.B. in Mundharmonikas zur Tonerzeugung eingesetzt werden. Die Vorrichtung (2), die auch als Atemtrainer bezeichnet werden kann, ist damit unabhängig vom Ort und von äusseren Energiequellen einsetzbar und wegen seines geringen Platzbedarfs leicht transportierbar.

## Beschreibung

Die vorliegende Erfindung bezieht sich auf eine Vorrichtung zur Selbstkontrolle der Atemtätigkeit gemäss Oberbegriff des Anspruchs 1.

Der Effekt der Rückkoppelung der eigenen Atmung durch akustische oder optische Signale ist gut untersucht und kann helfen, eine rasche, bewusste Entspannung zu erreichen.

Die sich im Handel befindlichen Geräte messen die Atmung, z.B. mittels Dehnungsstreifen am Thorax oder Thermodifferenz des Atemstromes an der Nase. Ein- und Ausatmung werden dann elektronisch umgewandelt und mittels eines akustischen oder optischen Signals über eine Brille oder einen Kopfhörer rückgekoppelt. Die damit erzielte bewusste Empfindung der eigenen Atmung kann helfen, eine rasche Entspannung und Beruhigung zu erwirken. Die sich auf dem Markt befindlichen Geräte sind im allgemeinen technisch relativ aufwendig, abhängig von einer Stromquelle und einzelne recht teuer.

Eine Aufgabe der vorliegenden Erfindung ist es, eine Vorrichtung anzugeben, die wenigstens einen der oben genannten Nachteile vermeidet und sich bevorzugt dadurch auszeichnet, dass sie einfacher verwendbar ist.

Eine derartige Vorrichtung ist im Anspruch 1 angegeben. Die weiteren Ansprüche enthalten bevorzugte Ausführungsformen.

Die erfindungsgemässe Vorrichtung zeichnet sich also dadurch aus, dass der Einatem- und/oder Ausatemluftstrom wenigstens einer Nasenöffnung, gegebenenfalls auch des Munds, durch einen Klangerzeuger geleitet wird. Der Klangerzeuger erzeugt daraus ein Signal, z.B. einen Ton, der bevorzugt qualitativ, z.B. in der Lautstärke, von der Atemintensität abhängig ist.

Das Signal wird bevorzugt direkt durch geeignete Übertragungsmittel, z.B. durch Stethoskop-Leitungen, auf ein Sinnesorgan der jeweiligen Person übertragen, z.B. das Ohr. Im Rahmen der Erfindung ist bei den Signalerzeugern an derartige zu denken, die entweder ohne externe Energiequellen auskommen oder allenfalls derartig geringen Energiebedarf haben, dass er aus der Umgebung gedeckt werden kann, z.B. aus Licht oder einer Wärmedifferenz. Allenfalls könnte auch eine in der Bauform kleine Energiequelle, z.B. eine Knopfzelle, in Betracht kommen. Die im wesentlichen gleichen Anforderungen sind auch an die Übertragungsmittel des Signals auf die Sinnesorgane zu stellen.

Die Erfindung soll weiter an Ausführungsbeispielen erläutert werden. Die Figuren zeigen:
- Fig. 1:: Eine Skizze einer erfindungsgemässen Vorrichtung in der Anwendung;
- Fig. 2:: eine räumliche Teildarstellung einer erfindungsgemässen Vorrichtung;
- Fig. 3:: eine Darstellung wie in Fig. 2 einer anderen erfindungsgemässen Vorrichtung.

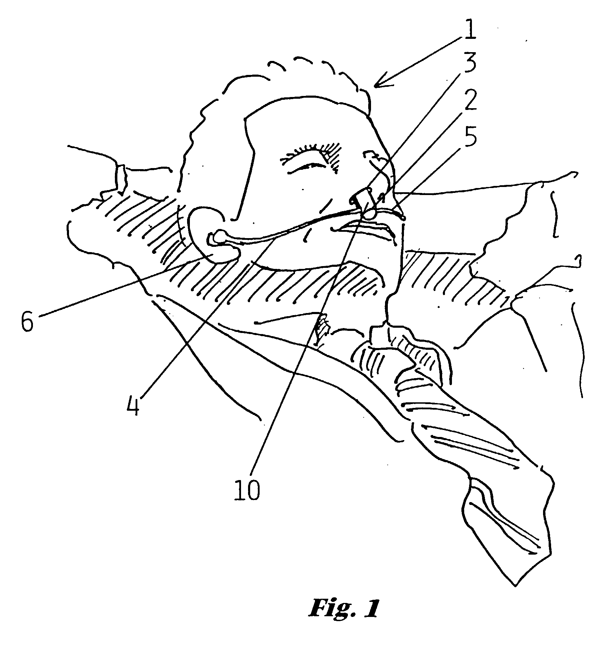

Fig. 1 zeigt eine Person 1, die die erfindungsgemässe Vorrichtung 2, im weiteren "Atemtrainer" genannt, in ihre Nasenöffnung 3 eingesetzt hat. Vom Atemtrainer 2 gehen ein rechter und ein linker Schlauch 4 bzw. 5 zu dem jeweiligen Ohr 6 der Person 1.

Das Gerät erzeugt ein akustisches Signal, indem der Atemstrom an der Nasenöffnung zur direkten physikalischen Tongeneration verwendet wird. Konkret verwenden wir den Luftstrom an der Nasenöffnung, welcher über einen Tonerzeuger in der Art einer Mundharmonika geleitet wird. Denkbar ist auch die Verwendung des Luftstromes am Mund bei Mundatmung. Das so erzeugte akustische Signal wird durch das feine Schlauchsystem 4, 5 direkt zum Ohr des Anwenders geleitet.

Das Gerät zeichnet sich dadurch aus, dass es klein und handlich ist, überall hin mitgenommen und jederzeit auch unabhängig von einer elektrischen Stromquelle angewendet werden kann.

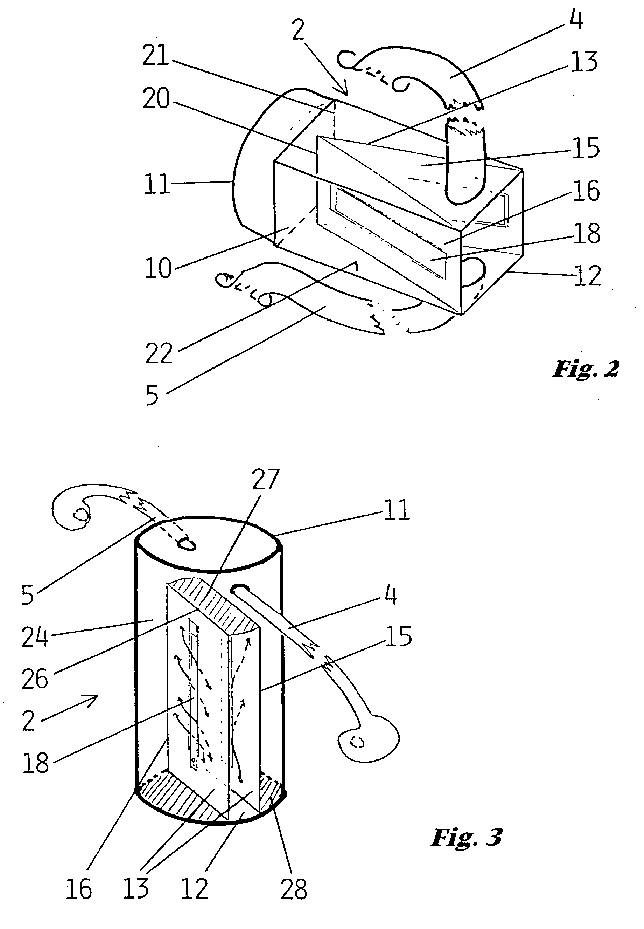

Fig. 2 und 3 zeigen schematisch zwei mögliche Ausführungsformen eines Atemtrainers. Die erste Ausführungsform gemäss Fig. 2 besteht im wesentlichen aus einem Gehäuse 10 mit einer Austrittsöffnung 12 und einer Eintrittsöffnung 11. Die Eintrittsöffnung 11 ist derart gestaltet und aus einem solchen Material gefertigt (z.B. ein nachgiebiges Silikonmaterial), dass dieses Ende des Gehäuses 10 wie oben beschrieben in eine Nasenöffnung 3 eingeführt werden kann. Wichtig ist dabei, dass zum einen ein genügend fester Halt in der Nasenöffnung erreicht wird, so dass der Atemluftstrom zu einem genügend grossen Teil durch den Atemtrainer 2 hindurchgeleitet wird und der Atemtrainer 2 insbesondere beim Ausatmen nicht aus der Nase ausgestossen wird, und andererseits durch Form und Material verhindert wird, dass das Vorhandensein des Atemtrainers 2 in einer Nasenöffnung 3 ein unangenehmes, störendes Tragegefühl hervorruft.

Im Inneren des Gehäuses 10 ist der Klangerzeuger 13 angeordnet. Er besteht aus einer V-förmigen Anordnung von zwei Stimmblättchen, wie sie z.B. für Stimmwerkzeug für Musikinstrumente, aber auch als Tonerzeuger in z.B. Mundharmonikas bekannt sind. Diese Blättchen 15, 16 wurden ausgewählt, da ihre Zungen 18 bereits bei dem geringen zur Verfügung stehenden Luftstrom zur Schwingung angeregt werden. Des weiteren zeichnen sich diese Blättchen auch dadurch aus, dass sie nur durch Luftstrom in einer bestimmten Richtung zur Tonerzeugung anregbar sind.

Die V-förmige Anordnung der Blättchen 15, 16 in Verbindung mit dem rechteckigen Innenquerschnitt des Gehäuses 10 erlaubt es auf einfache Art, den gesamten Luftstrom durch den Klangerzeuger hindurchzuleiten. Dabei ist ein Blättchen 15 so angeordnet, dass es nur beim Einatmen einen Ton erzeugt, während das andere Blättchen 16 nur auf den Ausatemluftstrom anspricht. Die V-förmige Anordnung kann eine einteilige, geknickte Ausführung eines doppelten Blättchens sein, kann aber auch aus zwei Blättchen zusammengesetzt sein. Im letzteren Fall ist, bevorzugt im Knick 20, eine Abdichtung, z.B. aus Silikonmasse, vorzusehen. Seitlich, d.h. in der oberen und unteren Seitenwand 21 bzw. 22 können Nuten vorgesehen werden, in die die Blättchen 15, 16 eingeschoben sind. Denkbar ist aber auch, den Klangerzeuger einfach in das Gehäuse 10 einzusetzen und darin mit Klebstoff, Dichtmasse oder auf eine andere, an sich bekannte Art zu befestigen.

Nahe der Austrittsöffnung 12 gehen die beiden Hörschläuche 4, 5 ab. Bei dieser Ausführungsform hört also jedes der beiden Ohren sowohl den Ton beim Einatmen wie auch beim Ausatmen. Zur besseren Atemkontrolle erzeugen hier die beiden Blättchen 15, 16 deutlich verschiedene Töne.

Bei der Ausführungsform der Fig. 3 sind die beiden Blättchen 15, 16 parallel zueinander in einem zylindrischen Gehäuse 24 angeordnet, z.B. in entsprechende Nuten in der Wand des Gehäuses 24 eingesetzt. An den austrittsseitigen Enden 26 ist der Durchgang zwischen den Blättchen 15, 16 durch eine Platte 27 verschlossen. Am Eintritt 12 ist der Innenraum des Gehäuses 24 ausserhalb der beiden Blättchen 15, 16 durch Platten 28 verschlossen. Dadurch wird der gesamte Atemluftstrom durch das Gehäuse 24 durch die beiden Blättchen 15, 16 geleitet.

In der gezeigten Ausführungsform sind die beiden Schläuche 4, 5 relativ zu den Blättchen 15, 16 gleichwertig angeordnet, so dass bei Fig. 3 zu beiden Ohren der gleiche Klangeindruck geleitet wird. Durch eine um 90° verdrehte Anordnung der Luftschläuche 4, 5 gegenüber dem Klangerzeuger 13 ist es jedoch möglich, dass ein Ohr, z.B. das rechte, im wesentlichen den Ton beim Einatmen vernimmt, der durch das Einatemblättchen 15 erzeugt wird, während das andere Ohr im wesentlichen nur den Ton vernimmt, der durch das Ausatemblättchen 16 erzeugt wird. Dadurch ist es möglich, zusätzlich zu verschiedenen Tonlagen für Ein- und Ausatemluftstrom eine (virtuelle) räumliche Unterscheidungsmöglichkeit vorzusehen. Denkbar ist dabei auch, für das Ein- und Ausatmen Blättchen gleicher Tonhöhe vorzusehen und die Unterscheidung zwischen den beiden Vorgängen einzig durch die räumliche Trennung zu ermöglichen. Es könnte damit ein angenehmer Schaukel- bzw. Wiegeeindruck vermittelt werden, der das Wohlbefinden steigert.

Für die Massenfertigung bietet sich als Herstellungsverfahren der Kunststoffspritzguss für das Gehäuse 10, 24 an. Wegen der einfachen Form ist die Ausführung gemäss Fig. 3 dafür besonders geeignet. Insbesondere verlaufen auch die für die Blättchen 15, 16 vorzusehenden Führungsnuten in axialer Richtung. Des weiteren kann der gesamte Klangerzeuger 13 getrennt fertiggestellt werden und als solcher einfach in Längsrichtung in das Gehäuse 10 eingesetzt werden. Es kann sich eine Versiegelung anschliessen, denkbar ist aber auch, dass das Gehäuse 10 z.B. eine umlaufende Nut nahe der Eintrittsöffnung 12 aufweist, in die die Abdeckplatten 28 einschnappen, wodurch eine hinreichende Abdichtung erzielbar ist.

Aufgrund der vorangehenden Beschreibung sind dem Fachmann eine Vielzahl von Abwandlungen zugänglich, ohne den Bereich der Erfindung wie in den Ansprüchen definiert zu verlassen. Denkbar sind z.B. die folgenden Abwandlungen:
- Anstelle der Atemschläuche sind andere Tonübertragungsmittel denkbar, wie z.B. ein oder mehrere Mikrophone in Verbindung mit Kopfhörern. Bevorzugt dürfen es dabei solche sein, die ohne externe Energiequelle ein Signal übermitteln.
- Die Form des Gehäuses 10, 24 und die Anordnung des Klangerzeugers 13 darin kann abgewandelt werden, soweit der Hauptluftstrom jeweils durch den Klangerzeuger geleitet wird.
- Die Eintrittsöffnung 12 kann auch so ausgebildet sein, dass z.B. eine Aufnahme in den Mund möglich ist, um den Luftstrom bei Mundatmung für das Atemtraining verwenden zu können. Sie kann auch mit einem auswechselbaren Ansatzstück versehen sein, um aus hygienischen Gründen ein Auswechseln zu ermöglichen. Auch ein längerer Ansatz, im Extremfall als Schlauch, ist denkbar.
- Anstelle der Blättchen 15, 16 können andere Klangerzeuger eingesetzt werden, die bevorzugt allein durch Anregung durch den Atemluftstrom einen Ton oder Klang erzeugen. Die Klangerzeuger können auch auswechselbar sein, um individuelle Vorlieben für bestimmte Tonhöhen und/oder -arten berücksichtigen zu können.
- Die Eintrittsöffnung 12 kann auf verschiedenste Art realisiert werden, sie muss insbesondere nicht unbedingt gewährleisten, dass der gesamte Luftstrom von z.B. einer Nasenöffnung auch durch das Gehäuse 10 geleitet wird.

## Patentansprüche

1. Vorrichtung zur Atemschulung durch Rückkoppelung der Atemtätigkeit einer Person (1) auf eines ihrer Sinnesorgane (6), gekennzeichnet durch mindestens einen Generator (15, 16) für akustische Signale, wobei der Generator durch einen Luftstrom der Atmung zur Abgabe eines akustischen Signales anregbar ist, und gekennzeichnet durch Mittel (4, 5) zur Übertragung des akustischen Signales auf mindestens ein Sinnesorgan der Person, insbesondere ein Ohr (6), um der Person (1) einen deutlichen Sinneseindruck der eigenen Atemtätigkeit zu verschaffen.

2. Vorrichtung gemäss Anspruch 1, dadurch gekennzeichnet, dass die Klanggeneratoren (15, 16) ohne externe Energiequellen auskommen, bevorzugt Blättchen mit einer durch einen Luftstrom zur Schwingung anregbaren Zunge (18) sind.

3. Vorrichtung gemäss einem der Ansprüche 1 bis 2, dadurch gekennzeichnet, dass die Klanggeneratoren (15, 16) in einem Gehäuse (10; 24), das eine Leitung für Atemluft darstellt, angeordnet sind, wobei das Gehäuse an einer Eintrittsstelle (12) zur Aufnahme mit dem Mund und/oder bevorzugt in mindestens einer Nasenöffnung der Person (1) ausgebildet ist, um den durch die jeweilige Körperöffnung gehenden Atemluftstrom durch das Gehäuse (10; 24) zu leiten.

4. Vorrichtung gemäss Anspruch 3, dadurch gekennzeichnet, dass die Klanggeneratoren (15, 16) entweder allein oder mit zusätzlichen Verschlussteilen (27, 28) derart im Gehäuse (10; 24) angeordnet sind, dass der Durchgangsquerschnitt durch diese Teile im wesentlichen verschlossen ist, um den das Gehäuse (10; 24) durchstreifenden Atemluftstrom möglichst vollständig den Klanggeneratoren (15, 16) zuzuführen.

5. Vorrichtung gemäss einem der Ansprüche 1 bis 4, dadurch gekennzeichnet, dass die Verbindungsmittel (4, 5) im wesentlichen mit einem Schallübertragungsmedium, insbesondere Luft, gefüllte Leitungen mit in ein Ohr (6) einsetzbaren Endstücken sind.

6. Vorrichtung gemäss einem der Ansprüche 1 bis 5, dadurch gekennzeichnet, dass mindestens zwei Klanggeneratoren (15, 16) vorhanden sind, wobei ein erster Klanggenerator (15) durch Luftstrom in eine Richtung und ein zweiter (16) durch Luftstrom in eine andere, bevorzugt entgegengesetzte, Richtung entsprechend dem Ein- bzw. Ausatemluftstrom anregbar ist.

7. Vorrichtung gemäss Anspruch 6, dadurch gekennzeichnet, dass die Klanggeneratoren (15, 16) für unterschiedliche Richtungen des Luftstromes verschiedene akustische Signale erzeugen.

8. Vorrichtung gemäss einem der Ansprüche 1 bis 7, dadurch gekennzeichnet, dass von mindestens einem, bevorzugt allen Klanggeneratoren (15, 16) ein angenehm klingender, bevorzugt oberwellenarmer und insbesondere bevorzugt nahezu sinusförmiger, Ton erzeugbar ist.

9. Vorrichtung gemäss einem der Ansprüche 1 bis 8, dadurch gekennzeichnet, dass alle Verbindungsmittel (4, 5) die Signale der Klanggeneratoren (15, 16) in in etwa gleicher Intensität aufnehmen.

10. Vorrichtung gemäss einem der Ansprüche 1 bis 8, dadurch gekennzeichnet, dass die Aufnahmestelle der Verbindungsmittel (4, 5) für die Signale der Klanggeneratoren (15, 16) jeweils stärker auf einen zugeordneten Klanggenerator (15, 16) ausgerichtet, insbesondere näher angeordnet ist, so dass das Signal des jeweils zugeordneten Klanggenerators mit höherer Lautstärke vom Übertragungsmittel erfassbar ist, um den Eindruck einer räumlichen Verteilung der Klanggeneratoren zu erzeugen.

11. Vorrichtung gemäss einem der Ansprüche 1 bis 10, dadurch gekennzeichnet, dass das Gehäuse (10; 24) wenigstens an der zur Verbindung mit Nase und/oder Mund einer Person (1) vorgesehenen Eintrittsöffnung (12) aus nachgiebigem und bevorzugt physiologisch verträglichem Material, insbesondere gummielastischer Silikonmasse, besteht, um einen hohen Benutzungskomfort zu erzielen.
